(19) Europäisches Patentamt
European Patent Office
Office européen des brevets

(11) Numéro de publication : **0 110 142**
**B1**

(12) **FASCICULE DE BREVET EUROPÉEN**

(45) Date de publication du fascicule du brevet : **30.12.86**

(51) Int. Cl.[4] : **C 07 C 45/54**, C 07 C 49/597, C 07 C 49/623, C 07 C 69/145

(21) Numéro de dépôt : **83110749.5**

(22) Date de dépôt : **27.10.83**

(54) **Procédé pour la préparation de cyclopenténones alkylées et esters acétyléniques utiles en tant que produits de départ dans ladite préparation.**

(30) Priorité : **06.12.82 CH 7075/82**

(43) Date de publication de la demande : **13.06.84 Bulletin 84/24**

(45) Mention de la délivrance du brevet : **30.12.86 Bulletin 86/52**

(84) Etats contractants désignés : **CH DE FR GB LI NL**

(56) Documents cités :
**US-A- 3 057 888**
**HELVETICA CHIMICA ACTA, vol. 59, no. 135, fasc. 4, 1976, pages 1328-1332; H. STRICKLER et al.: "Zur Cylisierung von Dehydrolinalylacetat in Gegenwart von Zinkchlorid"**
**CHEMICAL ABSTRACTS, vol. 76, 1972, page 299, résumé no. 3345w, Columbus, Ohio, US; B.M. GAVRILOV et al.: "Allyl migration and steric isomers of 3-methyl-2-penten-4-yn-1-ol".**

(73) Titulaire : **FIRMENICH SA**
**1, route des Jeunes**
**CH-1211 Genève 8 (CH)**

(72) Inventeur : **Rautenstrauch, Valentin, Dr.**
**69, chemin de Saule**
**CH-1233 Bernex (CH)**

(74) Mandataire : **Salvadori, Giuseppe, Dr.**
**c/o Firmenich S.A. Case Postale 239**
**CH-1211 Genève 8 (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 110 142 B1

Jouve, 18, rue St-Denis, 75001 Paris, France

**Description**

Certaines cyclopenténones comportant une double liaison endocyclique et un substituant alkyle en position α ou β du groupe carbonyle représentent des matières premières très utiles pour la synthèse de produits destinés à l'industrie de la parfumerie et des arômes, voire même de stéroïdes. Fort nombreuses sont les méthodes décrites à ce jour pour leur préparation et, à cet effet, un aperçu général relatif à la 2-méthyl-2-cyclopenténone a été présenté dans un article récent publié par R.L. Funk et K.P.C. Vollhardt [voir Synthesis 1980, 118]. Pour la préparation d'autres cyclopenténones, voir les références présentées par Naef et al. [Helv. Chim. Acta, *61,* 2524 (1978)] ainsi que par T.L. Ho et al. [Chem. Ind. (London) 1982, 371-372], et McCurry et al. [J. Org. Chem. *39,* 2317-2319 (1974)]. La présente invention apporte une solution nouvelle et originale à ce problème. Elle a trait en particulier à un procédé pour la préparation d'une cyclopenténone de formule

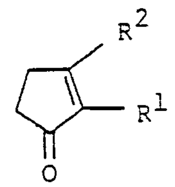
(I)

dans laquelle $R^1$ et $R^2$, pris séparément, représentent chacun un radical alkyle, de préférence de $C_1$ à $C_6$, ou un atome d'hydrogène, ou, pris conjointement, un polyméthylène, ou l'un d'entre eux représente un atome d'hydrogène et l'autre un radical alkyle tel que défini ci-dessus, caractérisé en ce qu'on effectue une cyclisation catalytique d'un ester de formule

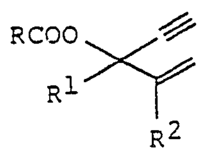
(II)

dans laquelle $R^1$ et $R^2$ ont le sens indiqué ci-dessus et R sert à désigner un radical alkyle inférieur de $C_1$ à $C_6$ ou un phényle, en présence d'un dérivé métallique de formule

$$MeX_2(R^3CN)_2 \qquad (III)$$

dans laquelle Me représente un atome de palladium ou de platine, $R^3$ définit un groupe alkyle inférieur de $C_1$ à $C_3$ ou un groupe phényle et X désigne un atome d'halogène, à une température comprise entre environ 50 et 100 °C, et qu'on isole ensuite le produit désiré de formule (I) du mélange réactionnel.

La réaction qui caractérise le procédé susmentionné est en soi nouvelle. Son déroulement est d'autant plus étonnant que l'art antérieur fait état du réarrangement d'acétates allyliques du type

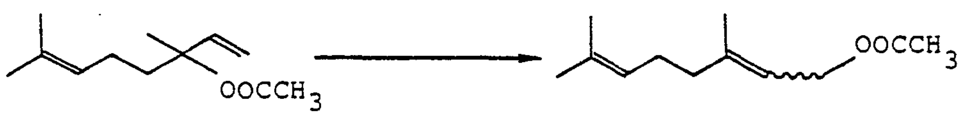

en présence précisément de catalyseurs constitués par des sels de palladium analogues à ceux de la formule (III) voir à cet effet :

a) P.M. Henry, J. Am. Chem. Soc. *94,* 5200 (1972) ;
b) L.E. Overman & F.M. Knoll, Tetrahedron Lett. 1979, 321 ;
c) P.A. Grieco, T. Takigawa, S.L. Bongers & H. Tanaka, J. Am. Chem. Soc. *102,* 7587 (1980) ;
d) P.A. Grieco, P.A. Tuthill & H.L. Sham, J. Org. Chem. *46,* 5005 (1981) ;
e) B.T. Golding, C. Pierpoint & R. Aneja, J. Chem. Soc. Chem. Comm. 1981, 1030).

La réaction peut être effectuée dans un solvant organique ou en absence de tout solvant et la présence de traces d'eau ne semble pas affecter le bon déroulement de la réaction. Parmi les solvants organiques utiles, il convient de citer tout particulièrement des éthers, tel le tétrahydrofuranne, les nitriles, tel l'acétonitrile, les hydrocarbures halogénés comme le chloroforme ou encore les alcools, tout spécialement le tert-butanol ou l'isopropanol. L'acétonitrile est utilisé de préférence. La réaction peut également être effectuée en présence d'un acide carboxylique. A titre d'exemple, on peut citer comme acide carboxylique utile l'acide acétique, propionique ou butyrique.

L'acide acétique est utilisé de préférence pour toute cyclisation des esters (II) ayant $R=CH_3$. Comme indiqué plus haut, la réaction a lieu en présence d'un dérivé du palladium ou du platine de formule (III). A titre préférentiel, on utilise les dérivés chlorés ou bromés (X = Cl ou Br). En tant que ligand de

l'halogénure de palladium, on emploie des nitriles, tel l'acétonitrile ou le benzonitrile, et l'on effectuera donc la réaction en présence de $MeX_2(CH_3CN)_2$ ou de $MeX_2(C_6H_5CN)_2$ [Me = Pd ou Pt].

La proportion du sel de palladium ou de platine dans le mélange de réaction est de l'ordre d'environ 5 à 10 moles % par rapport à la quantité molaire de l'ester acétylénique de formule (II) soumis à réaction.

Les temps de réaction sont relativement courts et bien entendu sont fonction de la température appliquée. Nous avons pu observer que des temps de l'ordre de 1-1 h 30 sont tout à fait suffisants pour promouvoir et compléter la conversion désirée. Dans un mode opératoire typique et préférentiel, on porte à la température choisie le mélange constitué par l'ester acétylénique (II) et le sel de palladium (III), dans un rapport molaire d'environ 20 : 1 respectivement. Après une heure environ, la réaction est arrêtée et le mélange obtenu est distillé sous pression réduite pour fournir la cyclopenténone (I) désirée.

Les esters de formule (II), utilisés comme produits de départ dans le procédé de l'invention, peuvent être aisément obtenus par estérification des alcools correspondants, lesquels sont soit des produits commerciaux, soit des produits accessibles par voie de synthèse.

Les schémas de réaction ci-dessous illustrent des méthodes de synthèse qui pourraient être appliquées à cet effet.

Schéma 1

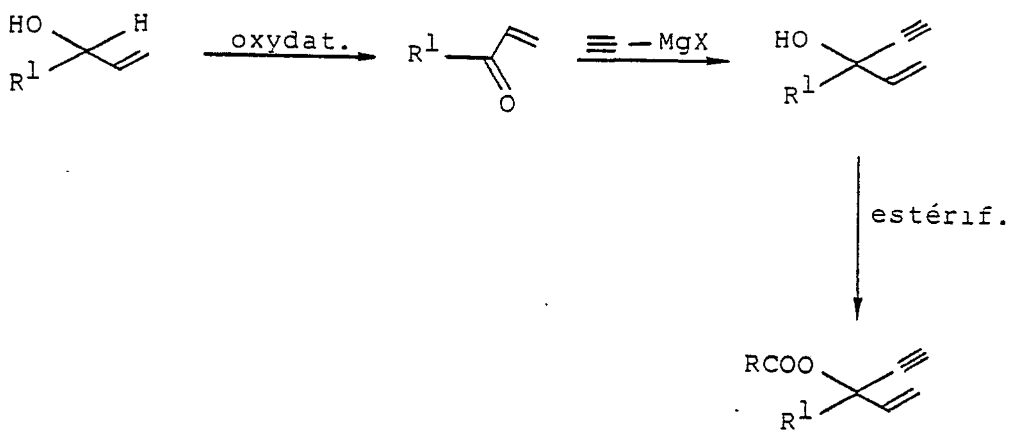


(IIa)

[voir par exemple : Org. Synth. Coll. Vol. *4*, 792 (1963) et Synthesis, 1976, 755]

Schéma 2

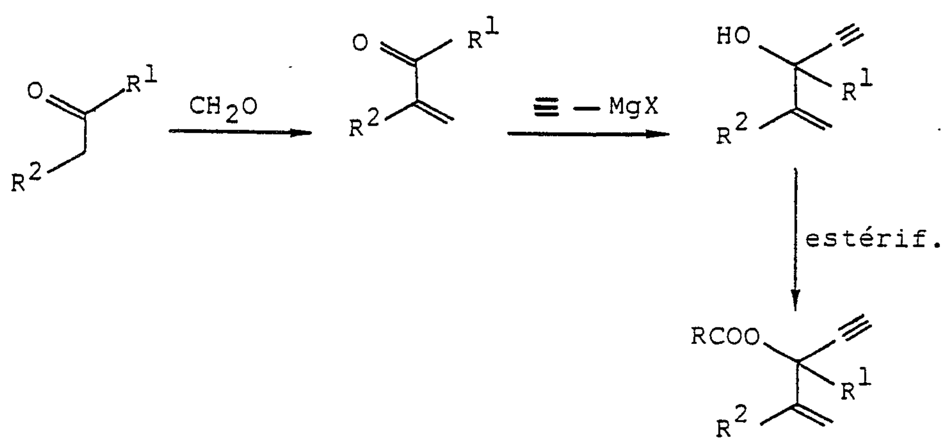


(IIb)

L'invention est illustrée de manière plus détaillée par l'un des exemples suivants dans lesquels les températures sont indiquées en degrés centigrades et les abréviations ont le sens usuel dans l'art.

Exemple 1

2-Méthyl-2-cyclopenténone

Un mélange constitué par 250 mg (1,81 mmole) d'acétate de 1-éthynyl-1-méthyl-2-propényle et 46 mg de $PdCl_2(CH_3CN)_2$ (0,18 mmole) dans 2,1 ml d'acétonitrile et 100 μl d'acide acétique glacial a été chauffé à 60° pendant 45 min. Un contrôle à l'aide de chromatographie en phase gazeuse a montré la conversion complète de l'acétate de départ. 1 Ml de méthanol et 1 cristal de 4-diméthylaminopyridine ont été ensuite

ajoutés au mélange réactionnel et le tout a été maintenu sous agitation pendant 30 min à température ambiante, puis il a été repris avec 10 ml d'un mélange de pentane/éther 1 : 1, une petite quantité de carbonate de potassium et agité pendant 1 h supplémentaire. Après filtration, les parties volatiles ont été évaporées et le résidu formé a été distillé à l'aide d'un four à boules pour fournir 101 mg du produit désiré (rend. 58 % ; pureté 96 %).

Exemple 2

2-Pentyl-2-cyclopenténone

Un mélange de 184 mg (0,95 mmole) d'acétate de 1-éthynyl-1-pentyl-2-propényle et 13 mg (0,05 mmole) de $PdCl_2(CH_3CN)_2$ dans 1,2 ml d'acétonitrile et 60 μl d'acide acétique glacial a été chauffé à 80° pendant 2 h. 1 Ml de méthanol et quelques cristaux de 4-diméthylaminopyridine ont été ajoutés au mélange réactionnel et le tout a été maintenu sous agitation à température ambiante pendant 45 min, puis il a été repris avec 10 ml d'un mélange de pentane/éther 1 : 1 et une petite quantité de carbonate de potassium. Après avoir été laissé sous agitation pendant 30 min supplémentaires, il a été filtré et évaporé. Le résidu ainsi formé a fourni par distillation à l'aide d'un appareil à boules 104 mg de distillat incolore constitué par le produit désiré ayant une pureté de 92 % (rend. 66 %).

En effectuant la réaction dans du toluène à la place d'acétonitrile et à une température d'environ 80°, on a obtenu le produit désiré, quoique avec un rendement inférieur.

L'acétate de 1-éthynyl-1-pentyl-2-propényle, utilisé comme produit de départ dans la méthode susmentionnée, a été préparé suivant un procédé analogue à celui décrit [Synthesis, 1976, 755]. Les caractères analytiques de l'ester étaient les suivants :

RMN (360 MHz) : 0,89 (3H, t, J = 7) ; 1,23-1,60 (6H, m) ; 1,74-1,84 ; 1,92-2,00 (1H, m) ; 2,05 (3H, s) ; 2,68 (1H, s) ; 5,28 (1H, d, J = 10,5) ; 5,58 (1H, d, J = 17) ; 5,88 (1H, d × d, J = 10,5 et 17) δ ppm.

SM : $M^+$ = 194 ; m/z : 123(21), 96(13), 95(11), 81(21), 78(11), 43(100) ;

IR : 1 750, 2 140, 3 290 $cm^{-1}$.

En suivant la même méthode que celle suivie pour la préparation de la 2-méthyl-2-cyclopenténone à l'exemple 1, on a préparé les produits que voici :

| produit | rendement (%) |
|---|---|
| 3-penthyl-2-cyclopenténone | 66 |
| 3-hexyl-2-cyclopenténone | 63 |
| 13-oxo-bicyclo [10.3.0] pentadéc-1(12)-ène | 76-89 |

Les caractères analytiques des acétates acétyléniques, utilisés comme produits de départ pour la préparation des produits ci-dessus mentionnés, étaient les suivants :

acétate de 1-éthynyl-2-pentyl-2-propényle

RMN (360 MHz) : 0,90 (3H, t, J = 7,0) ; 1,25-1,37 (4H, m) ; 1,45-1,58 (2H, m) ; 2,12 (3H, s) ; 2,09-2,23 (2H, m) ; 2,54 (1H, d, J = 2,2) ; 5,05, 5,34, 5,85 (3 × 1H, large s) δ ppm ;

SM : $M^+$ = 194 (< 0,1) ; m/z : 105(33), 95(27), 91(52), 79(26), 77(28), 43(100), 41(36).

acétate de 1-éthynyl-2-hexyl-2-propényle

RMN (360 MHz) : 0,90 (3H, t, J = 7,5) ; 1,25-1,39 (6H, m) ; 1,44-1,56 (2H m) ; 2,12 (3H, s) ; 2,09-2,23 (2H, m) ; 2,54 (1H, d, J = 1,8) ; 5,05, 5,34, 5,85 (3 × 1H, large s) δ ppm ;

MS : $M^+$ = 208 (< 0,1) ; m/z : 105(24), 95(16), 91(43), 79(20), 77(18), 43(100), 41(34).

acétate de 1-éthynyl-2-méthylène-cyclododécyle

RMN (360 MHz) : 1,16-1,60 (14H, m) ; 1,76-2,04 (4H, m) ; 2,06 (3H, s) ; 2,14-2,26 (2H, m) ; 2,72 (1H, s) ; 5,16 (1H, large s) ; 5,63 (1H, d, J = 2,2) δ ppm ;

SM : $M^+$ = 262 (< 0,1) ; m/z : 95(22), 91(28), 79(19), 67(19), 55(26), 43(100), 41(30) ;

IR : 2 110, 1 740 $cm^{-1}$ ;

F.66-68°.

**Revendications**

1. Procédé pour la préparation d'une cyclopenténone de formule

(Voir schéma p. 5)

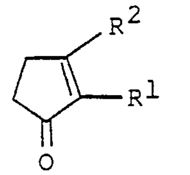

(I)

dans laquelle $R^1$ et $R^2$, pris séparément, représentent chacun un radical alkyle, de préférence de $C_1$ à $C_6$, ou un atome d'hydrogène, ou, pris conjointement, un polyméthylène, ou l'un d'entre eux représente un atome d'hydrogène et l'autre un radical alkyle tel que défini ci-dessus, caractérisé en ce qu'on effectue une cyclisation catalytique d'un ester de formule

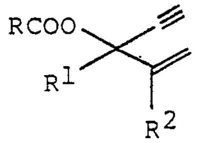

(II)

dans laquelle $R^1$ et $R^2$ ont le sens indiqué ci-dessus et R sert à désigner un radical alkyle inférieur de $C_1$ à $C_6$ ou un phényle, en présence d'un dérivé métallique de formule

$$MeX_2(R_3CN)_2 \quad (III)$$

dans laquelle Me représente un atome de palladium ou de platine, $R^3$ définit un groupe alkyle inférieur de $C_1$ à $C_3$ ou un groupe phényle et X désigne un atome d'halogène, à une température comprise entre environ 50 et 100 °C, et qu'on isole ensuite le produit désiré de formule (I) du mélange réactionnel.

2. Procédé suivant la revendication 1, caractérisé en ce que la réaction est effectuée dans un solvant organique.

3. Procédé suivant la revendication 1, caractérisé en ce qu'on utilise comme solvant organique l'acétonitrile ou l'acide acétique, ou un mélange des deux.

4. Procédé suivant la revendication 1, caractérisé en ce qu'on utilise comme composé de formule (II) l'acétate de 1-éthynyl-1-méthyl-2-propényle et comme composé de formule (III) le $PdCl_2(CH_3CN)_2$ et que l'on obtient la 2-méthyl-2-cyclopenténone.

5. Procédé suivant la revendication 1, caractérisé en ce qu'on utilise comme composé de formule (II) l'acétate de 1-éthynyl-1-pentyl-2-propényle et comme composé de formule (III) le $PdCl_2(CH_3CN)_2$ et que l'on obtient la 2-pentyl-2-cyclopenténone.

6. Procédé suivant la revendication 1, caractérisé en ce qu'on utilise comme composé de formule (II) l'acétate de 1-éthynyl-2-pentyl-2-propényle et comme composé de formule (III) le $PdCl_2(CH_3CN)_2$ et que l'on obtient la 3-pentyl-2-cyclopenténone.

7. Procédé suivant la revendication 1, caractérisé en ce qu'on utilise comme composé de formule (II) l'acétate de 1-éthynyl-2-hexyl-2-propényle et comme composé de formule (III) le $PdCl_2(CH_3CN)_2$ et que l'on obtient la 3-hexyl-2-cyclopenténone.

8. Procédé suivant la revendication 1, caractérisé en ce qu'on utilise comme composé de formule (II) l'acétate de 1-éthynyl-2-méthylène-cyclododécyle et comme composé de formule (III) le $PdCl_2(CH_3CN)_2$ et que l'on obtient le 13-oxo-bicyclo [10.3.0] pentadéc-1(12)-ène.

9. Ester de formule (II) selon la revendication 1 appartenant au groupe que voici :
acétate de 1-éthynyl-2-pentyl-2-propényle,
acétate de 1-éthynyl-2-hexyl-2-propényle,
acétate de 1-éthynyl-2-méthylène-cyclododécyle et
acétate de 1-éthynyl-1-pentyl-2-propényle.

**Claims**

1. Process for the preparation of a cyclopentenone of formula

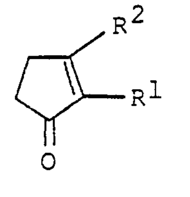

(I)

wherein each of $R^1$ and $R^2$, taken separately, represents an alkyl radical, preferably of $C_1$ to $C_6$, or a hydrogen atom, or, taken together, a polymethylene, or one of them represents a hydrogen atom and the other an alkyl radical as indicated above, characterized in that an ester of formula

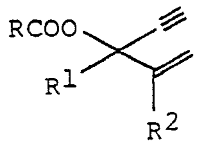

(II)

wherein $R^1$ and $R^2$ have the meaning indicated above and R designates a $C_1$ to $C_6$ lower alkyl radical or a phenyl, is catalytically cyclised in the presence of a metal derivative of formula

$$MeX_2(R^3CN)_2 \qquad (III)$$

wherein Me represents a palladium or a platinum atom, $R^3$ designates a $C_1$ to $C_3$ lower alkyl radical or a phenyl group and X stands for a halogen atom, at a temperature of between about 50 and 100 °C, and that the desired product of formula (I) is then isolated from the reaction mixture.

2. Process according to claim 1, characterized in that the reaction is effected in an organic solvent.

3. Process according to claim 1, characterized in that acetonitrile or acetic acid, or a mixture of the two, are used as organic solvent.

4. Process according to claim 1, characterized in that there is used as compound of formula (II) 1-ethynyl-1-methyl-2-propenyl acetate and as a compound of formula (III) $PdCl_2(CH_3CN)_2$ and there is obtained 2-methyl-2-cyclopentenone.

5. Process according to claim 1, characterized in that there is used as compound of formula (II) 1-ethynyl-1-pentyl-2-propenyl acetate and as a compound of formula (III) $PdCl_2(CH_3CN)_2$ and there is obtained 2-pentyl-2-cyclopentenone.

6. Process according to claim 1, characterized in that there is used as compound of formula (II) 1-ethynyl-2-pentyl-2-propenyl acetate and as a compound of formula (III) $PdCl_2(CH_3CN)_2$ and there is obtained 3-pentyl-2-cyclopentenone.

7. Process according to claim 1, characterized in that there is used as compound of formula (II) 1-ethynyl-2-hexyl-2-propenyl acetate and as a compound of formula (III) $PdCl_2(CH_3CN)_2$ and there is obtained 3-hexyl-2-cyclopentenone.

8. Process according to claim 1, characterized in that there is used as compound of formula (II) 1-ethynyl-2-methylene-cyclododecyl acetate and as a compound of formula (III) $PdCl_2(CH_3CN)_2$ and there is obtained 13-oxo-bicyclo [10.3.0] pentadec-1(12)-ene.

9. Ester of formula (II) according to claim 1 belonging to the following group :
1-ethynyl-2-pentyl-2-propenyl acetate
1-ethynyl-2-hexyl-2-propenyl acetate
1-ethynyl-2-methylene-cyclododecyl acetate and
1-ethynyl-1-pentyl-2-propenyl acetate.

**Patentansprüche**

1. Verfahren zur Herstellung eines Cyclopentanons der Formel

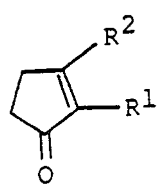

(I)

in welcher die Symbole $R^1$ und $R^2$, wenn sie getrennt genommen werden, einen Alkyl Rest, vorzugsweise von $C_1$ bis $C_6$, oder ein Wasserstoffatom bezeichnen, oder, wenn sie zusammen genommen werden, ein Polymethylen darstellen, oder eines der Symbole ein Wasserstoffatom und das andere einen wie obenangegebenen Alkyl Rest bedeutet,
dadurch gekennzeichnet, dass man ein Ester der Formel

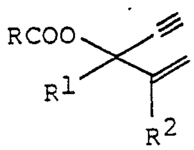

(II)

worin $R^1$ und $R^2$ die obenangegebene Bedeutung haben und R einen niedrigen $C_1$ bis $C_6$ Alkyl Rest oder ein Phenyl darstellt, bei einer Temperatur zwischen ungefähr 50 und 100 °C in Anwesenheit eines Metall

Derivats der Formel

$$MeX_2(R^3CN)_2 \quad (III)$$

in welcher Me ein Palladium- oder ein Platinatom bezeichnet, $R^3$ einen niedrigen $C_1$ bis $C_3$ Alkyl- oder Phenyl Rest darstellt und X ein Halogenatom ist, katalytisch cyclisiert, und das gewünschte Produkt der Formel (I) aus der Reaktionsmischung isoliert.

2. Verfahren gemäss Anspruch 1, dadurch gekennzeichnet, dass die Reaktion in einem organischen Lösungsmittel durchgeführt wird.

3. Verfahren gemäss Anspruch 1, dadurch gekennzeichnet, dass man Acetonitril oder Essigsäure, oder eine Mischung der beiden, als organisches Lösungsmittel verwendet.

4. Verfahren gemäss Anspruch 1, dadurch gekennzeichnet, dass man als Verbindung der Formel (II) 1-Ethynyl-1-methyl-2-propenylacetat, und als Verbindung der Formel (III) $PdCl_2(CH_3CN)_2$, verwendet, um 2-Methyl-2-cyclopentenon zu erhalten.

5. Verfahren gemäss Anspruch 1, dadurch gekennzeichnet, dass man als Verbindung der Formel (II) 1-Ethynyl-1-pentyl-2-propenylacetat, und als Verbindung der Formel (III) $PdCl_2(CH_3CN)_2$, verwendet, um 2-Pentyl-2-cyclopentenon zu erhalten.

6. Verfahren gemäss Anspruch 1, dadurch gekennzeichnet, dass man als Verbindung der Formel (II) 1-Ethynyl-2-pentyl-2-propenylacetat, und als Verbindung der Formel (III) $PdCl_2(CH_3CN)_2$, verwendet, um 3-Pentyl-2-cyclopentenon zu erhalten.

7. Verfahren gemäss Anspruch 1, dadurch gekennzeichnet, dass man als Verbindung der Formel (II) 1-Ethynyl-2-hexyl-2-propenylacetat, und als Verbindung der Formel (III) $PdCl_2(CH_3CN)_2$, verwendet, um 3-Hexyl-2-cyclopentenon zu erhalten.

8. Verfahren gemäss Anspruch 1, dadurch gekennzeichnet, dass man als Verbindung der Formel (II) 1-Ethynyl-2-methylen-cyclododecylacetat, und als Verbindung der Formel (III) $PdCl_2(CH_3CN)_2$, verwendet, um 13-Oxo-bicyclo [10.3.0] pentadec-1(12)-en zu erhalten.

9. Ein Ester der Formel (II), gemäss Anspruch 1, der zur folgenden Gruppe gehört :
1-Ethynyl-1-pentyl-2-propenylacetat,
1-Ethynyl-2-hexyl-2-propenylacetat,
1-Ethynyl-2-methylen-cyclododecylacetat und
1-Ethynyl-1-pentyl-2-propenylacetat.